# EUROPEAN PATENT APPLICATION

(11) **EP 3 400 968 A1**
(43) Date of publication of application: **14.11.2018**
(21) Application number: 17170043.8
(22) Date of filing: 09.05.2017
(51) Int. Cl.: A61L 2/06

(54) **AN APPARATUS AND A METHOD OF STERILIZING AND DRYING AT LEAST ONE ARTICLE**

(71) Applicant: Koninklijke Philips N.V., 5656 AE Eindhoven (NL)
(72) Inventor: ZHANG, Wei (Wayne), 5656 AE Eindhoven (NL)
(74) Representative: de Haan, Poul Erik

(57) **Abstract**

An apparatus (1) for sterilizing and drying at least one article (2, 3) comprises a barrel (10) configured to receive and accommodate said at least one article (2, 3); at least one heater (21, 22) configured to heat the air that is present inside the barrel (10) to a temperature that is in a range of 80°C to 100°C; and at least one air flow generator (30) that is at least partially arranged inside the barrel (10), the at least one air flow generator (30) being configured to cause the air that is present inside the barrel (10) to circulate within the barrel (10). Consequently, the apparatus (1) is configured to sterilize and dry at least one article (2, 3) only by exposing said at least one article (2, 3) to hot air inside the barrel (10).

## Description

### FIELD OF THE INVENTION

The invention relates to an apparatus and a method of sterilizing and drying at least one article.

### BACKGROUND OF THE INVENTION

An apparatus for sterilizing and drying at least one article is known in practice, and may be suitable for domestic use. Examples of articles that may need sterilizing and drying include baby bottles and accessories, tableware, and textile such as clothing, towels and handkerchiefs. In many cases, a sterilizing action is performed by means of ultraviolet light or steam, whereas a drying action is performed by means of infrared air and/or hot air. In the field of sterilizing and drying baby bottles and accessories, for example, it is known to have an apparatus that is adapted to use steam for sterilizing the bottles and accessories. However, a disadvantage of such an apparatus is that scale is deposited on various components of the apparatus, so that a user needs to subject the apparatus to a descaling action on a regular basis. Another disadvantage resides in the fact that a drying action takes quite a long time in view of the fact that moisture is added to the bottles and accessories during the preceding sterilizing action. When it comes to the drying function of the apparatus, it is known to use a fan to drive air through a heater for the purpose of generating hot air that is supplied to the bottles and accessories. The air is drawn in from the environment of the apparatus, and something like a mesh or filter is needed to prevent dust and other contaminations from reaching the inside of the apparatus. A disadvantage of this aspect of the known apparatus is that a user needs to clean or replace the mesh or filter on a regular basis.

### SUMMARY OF THE INVENTION

It is an object of the invention to provide an apparatus that is suitable for achieving sterilizing and drying results of at least one article that are at least as good as known in the art, while at the same time alleviating the above-mentioned problems that are normally associated with such an apparatus.

In view of the foregoing, the invention provides an apparatus for sterilizing and drying at least one article, comprising: a barrel configured to receive and accommodate said at least one article; at least one heater configured to heat air that is present inside the barrel to a temperature that is in a range of 80°C to 100°C; and at least one air flow generator that is at least partially arranged inside the barrel, the at least one air flow generator being configured to cause the air that is present inside the barrel to circulate within the barrel.

According to the invention, it is possible to only use air in the apparatus, for a combined purpose of sterilizing and drying. On the basis of the fact that the at least one heater of the apparatus is operable to heat air that is present inside the barrel to a temperature that is in a range of 80°C to 100°C, a sterilizing and drying action takes place at such a temperature and yields good results as a consequence thereof. The apparatus according to the invention is configured to perform a sterilizing and drying action on at least one article only by exposing said at least one article to hot air inside the barrel. The air is not drawn in from outside of the apparatus. Instead, the air that is present inside the barrel is circulated. Thus, second contamination effects are avoided without a need for a mesh or filter in the apparatus according to the invention. Also, there is nothing that adds to the moisture level prevailing inside the barrel, so that the drying action only takes a minimal amount of time. Scaling problems do not occur and there is no need for a user to perform a descaling operation on the apparatus.

In a practical embodiment, the apparatus according to the invention comprises a controlling system configured to control the at least one heater and the at least one air flow generator for sterilizing and drying at least one article. Such a controlling system may particularly be configured to operate the apparatus for performing a sterilizing and drying action involving an active state of both the at least one heater and the at least one air flow generator. Furthermore, the controlling system may include a timer, in which case a knob or the like may be provided on the apparatus for enabling a user to initiate a sterilizing and drying action and possibly also setting a duration of such an action. It is also possible for the controlling system to include a temperature sensor, which may be located at any suitable position in the apparatus, which position may be inside the barrel or at an outer surface of the barrel, for example. In any case, the temperature sensor may be used in a process of keeping the temperature of the air that is present inside the barrel well within the range of 80°C to 100°C during a sterilizing and drying action.

The controlling system may be further configured to control the at least one air flow generator for cooling the at least one article after the sterilizing and drying. Hence, the controlling system is enabled to operate the apparatus for performing a forced cooling action involving an active state of only the at least one air flow generator. In that way, it is possible to perform a sterilizing and drying action followed by a fast and effective cooling action, wherein the at least one article is first exposed to a flow of hot air for the purpose of sterilizing and drying, and wherein the at least one article is subsequently exposed to a flow of air that is in a process of cooling down for the purpose of cooling down to a temperature at which it is safe for a user to take hold of the at least one article. The duration of the cooling action may be determined by means of a timer, but it is also possible for the controlling system to be configured to terminate the cooling action only after it is found that the temperature detected by the temperature sensor is below a reference temperature. The controlling system may furthermore include a memory in which the reference temperature and/or other relevant data relating to operation of the apparatus are stored.

Alternatively, it is possible to let the at least one article cool down in a natural way, without the apparatus being operated, particularly without air being circulated within the barrel.

In a practical embodiment of the apparatus according to the invention, the barrel is at least partially made of metal material, the at least one heater being attached to an outer surface of the barrel. In general, it is advantageous if the at least one heater is at a position for supplying heat to the metal material. For example, the barrel may be generally shaped like a cylinder having a circular periphery, wherein the at least one heater is arranged so as to at least partially encompass the barrel along the periphery thereof, at a level where the barrel comprises a ring of metal material. In any case, using metal allows for realizing effective heating of the air that is present inside the barrel while allowing for an arrangement in which the at least one heater does not need to be present inside the barrel. Hence, within the framework of the invention, it is possible to let the at least one heater perform the function of heating the air that is present inside the barrel in an indirect fashion, by supplying heat to the barrel, wherein the heat is transferred from the at least one heater to the air through the barrel.

In order for a user to be capable of reaching the inside of the barrel for the purposes of putting at least one article in place inside the barrel and removing at least one article from the barrel, it is practical if a lid is provided that can be put to a first position for closing the barrel and that can be put to a second position for allowing access to the barrel. The lid may comprise at least one venting hole, i.e. at least one venting hole may be arranged in the lid, which does not alter the fact that another position of at least one venting hole may be practical as well. For the sake of completeness, it is noted that a venting hole is a hole that serves for letting out moisture from the barrel during a sterilizing and drying action.

It is practical for the at least one air flow generator to comprise a fan and a motor for rotating the fan, the fan being arranged inside the barrel, and the motor being arranged outside of the barrel. In such a case, the fan may particularly be arranged in a bottom area of the barrel, and the motor may be arranged underneath the barrel. In respect of terms like "bottom" and "underneath" as used in the present text, it is noted that such terms are in no way limiting to the invention and should be understood on the basis of the assumption of a normal, operational orientation of the apparatus according to the invention.

It is practical for the barrel, the at least one heater and the at least one air flow generator to be accommodated inside a housing. Furthermore, it is practical for the apparatus according to the invention to comprise at least one structure configured to support at least one article inside the barrel. Such a structure may comprise a bracket or grid, for example.

The invention furthermore relates to a method of sterilizing and drying at least one article placed inside a barrel, comprising the steps of: heating air that is present inside the barrel to a temperature that is in a range of 80°C to 100°C; and during the heating step, circulating the air that is present inside the barrel within the barrel. As mentioned in the foregoing, a sterilizing and drying action is performed under the influence of hot air only, particularly under the influence of air that is present inside the barrel and that is heated to an appropriate temperature, i.e. temperature in the range as mentioned, without any air being taken in from outside of the barrel.

According to one aspect of the invention as already explained in the foregoing, it may be so that heating of air that is present inside the barrel is terminated earlier than circulating said air within the barrel, so that a sterilizing and drying action followed by a cooling action is realized.

The above-described and other aspects of the invention will be apparent from and elucidated with reference to the following detailed description of a practical embodiment of an apparatus that is designed to subject baby bottles and accessories to a sterilizing and drying action. The apparatus that is described is only one example of the many possibilities existing within the framework of the invention. In particular, the invention is not limited to apparatuses that are suitable to be used for sterilizing and drying baby bottles and accessories. Within the framework of the invention, it is also possible to have an apparatus that is particularly designed to subject articles of at least one other type to a sterilizing and drying action, examples of such articles including tableware, and textile such as clothing, towels and handkerchiefs, as mentioned in the foregoing.

### BRIEF DESCRIPTION OF THE DRAWINGS

The invention will now be explained in greater detail with reference to the figures, in which equal or similar parts are indicated by the same reference signs, and in which:
Figures 1 and 2 show two different perspective sectional views of an apparatus according to a practical embodiment of the invention, which apparatus is designed to subject baby bottles and accessories to a sterilizing and drying action, wherein figures 1 and 2 furthermore show a baby bottle as present inside the apparatus;
Figure 3 shows a perspective view of the apparatus; and
Figure 4 shows a diagrammatic view of the apparatus and a number of baby bottles and accessories as present inside the apparatus.

### DETAILED DESCRIPTION OF EMBODIMENTS

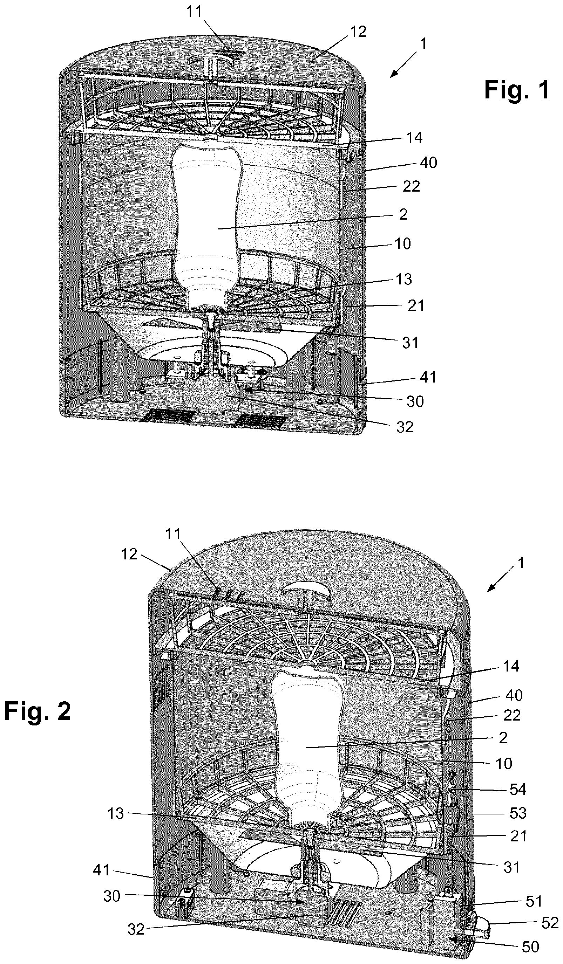

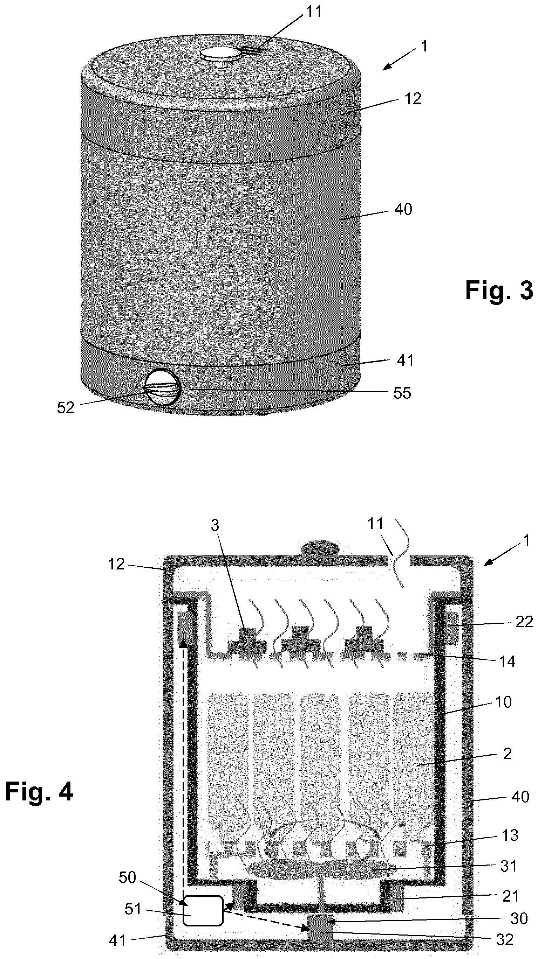

Figures 1-4 relate to a practical embodiment of the apparatus 1 according to the invention, and illustrate a normal, operational orientation of the apparatus 1. In the shown example, the apparatus 1 is particularly designed to be used for subjecting baby bottles 2 and accessories 3 to a sterilizing and drying action. Examples of accessories 3 include caps and teats. In figures 1 and 2, a single baby bottle 2 is shown, whereas in figure 4, as many as five baby bottles 2 are shown, along with three accessories 3. It will be understood that the invention is in no way limited to the intended use of the apparatus 1, not only when it comes to the type of articles to be subjected to a sterilizing and drying action, but also when it comes to the number of articles that can be treated inside the apparatus 1.

The apparatus 1 comprises a barrel 10 that is configured to receive and accommodate the baby bottles 2 and accessories 3, i.e. to serve as a container for containing articles 2, 3 to be sterilized and dried. In the shown example, the barrel 10 is fully closeable with the exception of a number of venting holes 11 that are designed to let out moisture from the barrel 10 during a sterilizing and drying action. The barrel 10 may have any suitable shape and size. It is practical for the barrel 10 to be generally shaped like a cup, having an open side at the top thereof. Furthermore, it is advantageous if the shape and size of the barrel 10 are chosen such as to allow the barrel 10 to accommodate a number of baby bottles 2 and a number of accessories 3 while at the same time not being too bulky and not including too much empty space. The number of any venting holes 11 is at least one. It is practical for such venting holes 11 to be large enough for letting out moisture from the barrel 10 and to be small enough to avoid an inflow of outside air in the barrel 10. In that respect, it is noted that the venting holes 11 may be realized as a series of elongated slits, as illustrated in figures 1-3.

The barrel 10 is openable and closeable by means of a lid 12 that is arranged and configured to cover the open side of the barrel 10 in one position, and to have the open side of the barrel 10 exposed in another position. The lid 12 may have a removable arrangement in the apparatus 1, but it is also possible for the lid 12 to be movably arranged in the apparatus 1, in which case the lid 12 may be mounted on one or more hinges so as to enable a hinging movement of the lid 12, for example.

In the shown example, the apparatus 1 is equipped with two heaters 21, 22, one heater being a bottom heater 21 that is arranged so as to encompass the barrel 10 at a bottom level of the barrel 10, and another heater being a top heater 22 that is arranged so as to encompass the barrel 10 at a top level of the barrel 10. For the purpose of allowing the barrel 10 to radiate heat that is supplied to the barrel 10 by the heaters 21, 22 during operation thereof to the air that is present inside the barrel 10, it is advantageous if the barrel 10 is made of metal material such as stainless steel, or at least comprises metal parts at the levels where the heaters 21, 22 are located.

It is practical for the apparatus 1 to comprise at least one structure for supporting baby bottles 2 and accessories 3 at a position inside the barrel 10. In this respect, figures 1, 2 and 4 illustrate the possibility of having a bottom grid 13 in a bottom area of the barrel 10 for supporting baby bottles 2 and a top grid 14 in a top area of the barrel 10 for supporting accessories 3. In the arrangement as shown, at least the top grid 14 is removably arranged in the apparatus 1 so as to allow a user to remove the top grid 14 in case it is desired to have access to the bottom grid 13 and any articles 2, 3 as may be present on the bottom grid 13. The bottom grid 13 may be removably arranged in the apparatus 1 as well, which may be advantageous in view of cleaning purposes.

Another notable component of the apparatus 1 according to the invention is an air flow generator 30. It is practical for the air flow generator 30 to comprise a fan 31 and a motor 32 for imposing a rotary movement on the fan 31, as is the case in the shown example. The fan 31 may be located at any suitable position inside the barrel 10 for circulating the air that is present inside the barrel 10. A position underneath the bottom grid 13 is a practical example of such position, all the more since at that position, the fan 31 is capable of directly supplying an air flow to inside a baby bottle 2 placed on the bottom grid 13 as well. In this respect, it is noted that the circulation of air is indicated in figure 4 by means of two curved arrows.

In the shown example, the motor 32 for driving the fan 31 is located outside of the barrel 10, underneath a bottom of the barrel 10. In that way, it is achieved that the motor 32 is kept outside of the area where hot air is present during a sterilizing and drying action. The fan 31 is connected to the motor 32 through a drive shaft 33 extending through the bottom of the barrel 10.

As shown in the figures, the apparatus 1 according to the invention may comprise a housing 40 for accommodating the various components mentioned in the foregoing, i.e. the barrel 10 including the grids 13, 14, the heaters 21, 22 and the air flow generator 30. The motor 32 of the air flow generator 30 is arranged in a bottom part 41 of the housing 40, in a space that is present between a bottom of the housing 40 and the bottom of the barrel 10. Besides the function of holding the various components, the housing 40 has a function of realizing heat insulation of the barrel 10.

The apparatus 1 according to the invention is intended to be an electrical appliance that is driven by electrical current. In conformity with what is known in the field of electrical appliances, the apparatus 1 comprises a controlling system 50 that is configured to control operation of the apparatus 1. Such a controlling system 50 may comprise a timer 51 and a timer knob 52 arranged at the outside of the housing 40 for allowing a user to put the timer 51 to an appropriate setting. In general, the controlling system 50 may comprise some type of controller and also an on/off switch and/or another type of user interface for allowing a user to at least activate the apparatus 1 and possibly provide the controller with information about a desired mode of operation in case the controller is programmed with various modes. In the case of the apparatus 1, the controlling system 50 is particularly designed to control operation of the heaters 21, 22 and the motor 32 of the air flow generator 30, as indicated in figure 4 by means of dashed arrows. The controlling system 50 may furthermore comprise a temperature sensor 53 for detecting a temperature at an appropriate position inside the barrel 10 or on an outer surface of the barrel 10, for the purpose of enabling temperature control in the apparatus 1, and also a thermal fuse 54 for avoiding overheating, as shown in figure 2. Figure 3 illustrates the possibility of having an indicator such as a light 55 on the apparatus 1 for providing a user with information about the operational status of the apparatus 1.

In the following, a description is given of how the apparatus 1 can be used and controlled for sterilizing and drying a baby bottle 2 and a cap 3. In a first instance, a user puts the lid 12 to an opened position and removes the top grid 14 from the apparatus 1, so that it is possible to place the baby bottle 2 on the bottom grid 13. Subsequently, the user puts the top grid 14 back in place in the apparatus 1 and places the cap 3 on the top grid 14. For the sake of completeness, it is noted that it is possible to omit the top grid 14 from the apparatus 1, in which case all articles to be subjected to a sterilizing and drying action are placed on the bottom grid 13. After everything has been put in place, the user puts the lid 12 to a closed position, i.e. a position as illustrated in the figures, in which the open side of the barrel 10 is covered.

When the user decides to initiate operation of the apparatus 1 and manipulates the timer knob 52, the controlling system 50 is activated to operate the heaters 21, 22 and the air flow generator 30. The heaters 21, 22 are activated to supply heat to the barrel 10, so that the barrel 10 heats up by heat conduction, and the temperature of the air that is present inside the barrel 10 increases under the influence of heat radiation from the barrel 10. In order to realize an effective sterilizing and drying action, the air may be heated to a temperature that is in a range of 80°C to 100°C. The fact is that such a temperature is high enough for killing bacteria and low enough for avoiding deformation of the articles 2, 3 to be subjected to the sterilizing and drying action. The temperature sensor 53 can be used to check an actual temperature prevailing inside the apparatus 1 and induce an appropriate controlling action, so that the heaters 21, 22 may be kept switched on as long as the temperature appears to be low, and the heaters 21, 22 may be operated at a low power level or switched off when the temperature appears to be sufficiently high. The air flow generator 30 is activated as well, so that the heat transfer is reinforced on the basis of the circulation of air that is induced. In the process, moisture escapes from the baby bottle 2 and the cap 3, and eventually from the barrel 10 through the venting holes 11, as indicated by curved lines in figure 4. All the time during the sterilizing and drying action, the user is informed of the fact that the apparatus 1 is in an active state by means of the light 55.

It is possible for the controlling system 50 to be designed to control the apparatus 1 to perform a sterilizing and drying action followed by a forced cooling action, wherein such a forced cooling action can be initiated when the sterilizing and drying action has been performed during a period of time that is long enough for that primary action to have good results. The heaters 21, 22 are then shut off while the air flow generator 30 is kept in the active state. Under the influence of the flow of air that is induced by the air flow generator 30, the baby bottle 2 and the cap 3 are cooled down fast and effectively, until the air flow generator 30 is shut off as well. The temperature sensor 53 can be used for detecting that a relevant temperature such as a temperature of the barrel wall is below a reference value at which it can be assumed that the baby bottle 2 and the cap 3 are at a temperature that is safe for a user. The reference value of the temperature may be 50°C, for example. The apparatus 1 can be equipped with a system for keeping the lid 12 locked in the closed position as long as the reference temperature has not yet been reached.

In case predetermined values are involved in the functioning of the controlling system 50, such values can be stored in a memory that is part of the controlling system 50, for one or various modes of operation in case the apparatus 1 is designed to allow a user to choose from various sterilizing and drying options. Examples of predetermined values include (i) a duration of the period during which the temperature of the air should be kept in the range of 80°C to 100°C for realizing an effective sterilizing and drying action and (ii) a reference temperature for determining a moment at which a forced cooling action can be terminated.

It will be clear to a person skilled in the art that the scope of the invention is not limited to the examples discussed in the foregoing, but that several amendments and modifications thereof are possible without deviating from the scope of the invention as defined in the attached claims. It is intended that the invention be construed as including all such amendments and modifications insofar they come within the scope of the claims or the equivalents thereof. While the invention has been illustrated and described in detail in the figures and the description, such illustration and description are to be considered illustrative or exemplary only, and not restrictive. The invention is not limited to the disclosed embodiments. The drawings are schematic, wherein details that are not required for understanding the invention may have been omitted, and not necessarily to scale.

Variations to the disclosed embodiments can be understood and effected by a person skilled in the art in practicing the claimed invention, from a study of the figures, the description and the attached claims. In the claims, the word "comprising" does not exclude other steps or elements, and the indefinite article "a" or "an" does not exclude a plurality. Any reference signs in the claims should not be construed as limiting the scope of the invention.

Elements and aspects discussed for or in relation with a particular embodiment may be suitably combined with elements and aspects of other embodiments, unless explicitly stated otherwise. Thus, the mere fact that certain measures are recited in mutually different dependent claims does not indicate that a combination of these measures cannot be used to advantage.

The term "comprise" as used in this text will be understood by a person skilled in the art as covering the term "consist of'. Hence, the term "comprise" may in respect of an embodiment mean "consist of", but may in another embodiment mean "contain/include at least the defined species and optionally one or more other species".

## Claims

1. An apparatus (1) for sterilizing and drying at least one article (2, 3), comprising:
- a barrel (10) configured to receive and accommodate said at least one article (2, 3);
- at least one heater (21, 22) configured to heat air that is present inside the barrel (10) to a temperature that is in a range of 80°C to 100°C; and
- at least one air flow generator (30) that is at least partially arranged inside the barrel (10), the at least one air flow generator (30) being configured to cause the air that is present inside the barrel (10) to circulate within the barrel (10).

2. The apparatus (1) according to claim 1, comprising a controlling system (50) configured to control the at least one heater (21, 22) and the at least one air flow generator (30) for sterilizing and drying at least one article (2, 3).

3. The apparatus (1) according to claim 2, wherein the controlling system (50) includes a timer (51).

4. The apparatus (1) according to claim 2 or 3, wherein the controlling system (50) includes a temperature sensor (53).

5. The apparatus (1) according to any of claims 2-4, wherein the controlling system (50) is further configured to control the at least one air flow generator (30) for cooling the at least one article (2, 3) after the sterilizing and drying.

6. The apparatus (1) according to claim 5 insofar as dependent on claim 4, wherein the controlling system (50) is configured to terminate the cooling action only after it is found that the temperature detected by the temperature sensor (53) is below a reference temperature.

7. The apparatus (1) according to any of claims 1-6, wherein the barrel (10) is at least partially made of metal material, the at least one heater (21, 22) being attached to an outer surface of the metal material.

8. The apparatus (1) according to any of claims 1-7, comprising a lid (12) that is movable between a first position for closing the barrel (10) and a second position for allowing access to the barrel (10).

9. The apparatus (1) according to claim 8, wherein the lid (12) comprises at least one venting hole (11).

10. The apparatus (1) according to any of claims 1-9, wherein the at least one air flow generator (30) comprises a fan (31) and a motor (32) for rotating the fan (31), the fan (31) being arranged inside the barrel (10), and the motor (32) being arranged outside of the barrel (10).

11. The apparatus (1) according to claim 10, wherein the fan (31) is arranged in a bottom area of the barrel (10), and wherein the motor (32) is arranged underneath the barrel (10).

12. The apparatus (1) according to any of claims 1-11, comprising a housing (40) for accommodating the barrel (10), the at least one heater (21, 22) and the at least one air flow generator (30).

13. The apparatus (1) according to any of claims 1-12, comprising at least one structure (13, 14) configured to support at least one article (2, 3) inside the barrel (10).

14. A method of sterilizing and drying at least one article (2, 3) placed inside a barrel (10), comprising steps of:
- heating air that is present inside the barrel (10) to a temperature that is in a range of 80°C to 100°C; and
- during the heating step, circulating the air that is present inside the barrel (10) within the barrel (10).

15. The method according to claim 14, wherein heating of air that is present inside the barrel (10) is terminated earlier than circulating said air within the barrel (10).
